(19) Europäisches Patentamt / European Patent Office / Office européen des brevets

(11) **EP 3 242 112 B1**

(12) **EUROPEAN PATENT SPECIFICATION**

(45) Date of publication and mention of the grant of the patent:
**18.09.2019 Bulletin 2019/38**

(51) Int Cl.:
***G01F 22/00*** *(2006.01)*  ***G01F 1/68*** *(2006.01)*
***A61B 5/00*** *(2006.01)*

(21) Application number: **17160730.2**

(22) Date of filing: **14.03.2017**

(54) **METHOD AND DEVICE FOR MEASUREMENT OF LIQUID FLOW RATE**

VERFAHREN UND VORRICHTUNG ZUR MESSUNG DER FLÜSSIGKEITSFLUSSRATE

PROCÉDÉ ET DISPOSITIF DE MESURE DE DÉBIT DE LIQUIDE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.05.2016 EP 16168351**

(43) Date of publication of application:
**08.11.2017 Bulletin 2017/45**

(73) Proprietor: **CSEM**
**Centre Suisse d'Electronique et de Microtechnique SA**
**2002 Neuchâtel (CH)**

(72) Inventors:
• **Schmid, David**
**7204 Untervaz (CH)**
• **Generelli, Silvia**
**7000 Chur (CH)**
• **Pereira, Fiona**
**Garland Close, SE16AY (GB)**

(74) Representative: **e-Patent SA**
**Rue Saint-Honoré 1**
**Boîte Postale CP 2510**
**2001 Neuchâtel (CH)**

(56) References cited:
**WO-A1-2017/019602  WO-A2-01/56436**
**US-A1- 2003 215 334**

• **XIAOZE DU ET AL: "Continuous micro liquid delivery by evaporation on a gradient-capillary microstructure surface", JOURNAL OF MICROMECHANICS & MICROENGINEERING, INSTITUTE OF PHYSICS PUBLISHING, BRISTOL, GB, vol. 21, no. 9, 29 July 2011 (2011-07-29), page 95004, XP020210103, ISSN: 0960-1317, DOI: 10.1088/0960-1317/21/9/095004**
• **NIE CHUAN ET AL: "A microfluidic device based on an evaporation-driven micropump", BIOMEDICAL MICRODEVICES, KLUWER, DORDRECHT, NL, vol. 17, no. 2, 25 March 2015 (2015-03-25) , pages 1-12, XP035499142, ISSN: 1387-2176, DOI: 10.1007/S10544-015-9948-7 [retrieved on 2015-03-25]**

## Description

## Technical Field

The present invention relates to the technical field of liquid measurements. More particularly, it relates to a method and a corresponding device for measuring relatively small liquid flow rates.

## State of the art

Measurement of small liquid volumes and/or flow rates involving relatively small amounts of liquid is important in, for instance, investigation of sweating, in microfluidic analysis, and in other roles.

The publication Closed-chamber transepidermal water loss measurement: microclimate, calibration and performance, Imhof et al, Int J Cosmet Sci. 2009 Apr;31(2):97-118, proposes a closed-chamber transdermal water loss measurement system. This system comprises an open-ended chamber which is placed on the skin of a subject so as to form a closed chamber, and comprises a relative humidity and temperature sensor situated inside the chamber. Measurements of the relative humidity and temperature as a function of time permit calculation of the sweating rate of the skin confined by the chamber, and by extrapolation the overall sweating rate of the person can be estimated.

However, since the chamber is closed, the air inside quickly saturates with water vapour from the person's sweat, and the accuracy of the measurement drops off rapidly. Long-term sweat rate measurements, e.g. extending over several hours or days, are thus difficult or impossible to perform accurately. Furthermore, this sensor cannot easily be adapted to measuring other liquid flow rates.

The document US2014/208824 describes a method of measuring small liquid volumes in microwell plates, which involves heating the liquid with a known quantity of thermal energy, and measuring the resulting temperature rise. From the energy applied and the temperature rise measured, the volume of liquid can be determined. However, since this method relies simply on measuring temperature differences, it presumes that evaporation of the sample is trivial and can thus be ignored. Indeed, the temperature changes used are from 1K to 30K for aqueous liquids and from 1K to 50K for oils, which are clearly chosen to minimise evaporation and to prevent sample damage or loss. In fact larger temperature changes are counter-indicated for microwell applications, since they will damage the biological sample whose volume is being measured by denaturing enzymes or killing cells. This method is thus essentially limited to the stated application, and is capable only of carrying out volumetric measurements.

Xiaoze Du et al, "Continuous micro liquid delivery by evaporation on a gradient-capillary microstructure surface", Journal of Microengineering, Institute of Physics Publishing, Bristol, GB, vol. 21, no.9 page 95004 (XP020210103) and patent application US 2003/215334 both describe evaporative microfluidic pumps in which a voltage is applied to a heating element so as to cause evaporation of liquid, which draws further liquid through the pump. Such pumps and their methods of operation allow obtaining a predetermined desired flow rate by varying the heating power, but they are completely unsuitable for measuring unknown flow rates of liquid.

WO 01/56436 describes an electric water heater in which the volume of water contained therein can be estimated based on the rate of temperature rise during heating. However, this arrangement is not suitable for measuring unknown flow rates.

Finally, WO2017/019602 and Nie Chuan et al, "A microfluidic device based on an evaporation-driven micropump", Biomedical Devices, Kluwer, Dordrecht, NL, vol. 17, no. 2, pages 1-12 (XP035499142) describe microfluidic devices for measurements relating to human sweat.

An aim of the invention is thus to propose a method for making flow rate measurements of liquids which overcomes at least partially the above-mentioned disadvantages of the prior art, and are hence suitable for a wide range of applications.

## Disclosure of the invention

More specifically, the invention relates to method for liquid measurements of unknown flow rates according to independent claim 1. This method comprises first providing a sensor comprising a surface arranged to receive a quantity of liquid, an electrical heating element arranged to heat said surface, and an arrangement for measuring a temperature of said surface.

Liquid is then received on said sensor surface.

The surface is heated by means of the electrical heating element at a known predetermined or measured rate while taking a plurality of temperature measurements of the surface as a function of time, i.e. at different, known times. This heating is carried out at an absolute temperature of at least 90% of the boiling point of said liquid under ambient pressure, so as to be able to heat the liquid at least to this temperature, if not higher. For instance, the surface which is in contact with the liquid is heated to at least this temperature.

On the basis of parameters relating to the electrical heating and the temperature measurements, a flow rate measurement of the unknown flow rate of the liquid is then derived, for instance by determining its flow rate from first principles related to the applied power, time, temperature, specific heat capacity and latent heat of vaporisation of the liquid, or alternatively by applying a previously-determined calibration curve.

As a result, the unknown flow-rate of the liquid on the surface of the sensor can easily and simply be determined, and since the sensor operates in an evaporative mode, the sensor is immediately ready to receive more liquid as the earlier liquid evaporates off. This evaporation can also advantageously be used to help draw fluid through an analysis sensor by creating a humidity gradient, thereby preventing the analysis sensor from clogging with analyte.

Advantageously, the electrical power source may be provided with a predetermined electrical current from a power source. By using a current source rather than a voltage source, the calculation of applied power is simplified if the electrical resistance of the heating element is known.

Advantageously, said temperature may be measured by measuring the electrical resistance of said electrical heating element. The electrical heating element thus serves not only as a source of heat, but also as its own arrangement for measuring temperature. This requires knowing the relationship between temperature and resistance of the electrical heating element, however it eliminates the requirement for a separate temperature-measuring element such as a thermistor. The electrical resistance can be measured by providing a known current to said electrical heating element while measuring the voltage drop thereacross, the resistance being calculated by Ohm's Law. This result can then be converted into temperature by means of a calibration curve, lookup table or similar.

Advantageously, the above-mentioned heating may be carried out as a series of heating pulses of predetermined duration, each preferably being sufficiently long to dry said surface. The flow rate of liquid can then be determined by these repeated measurements, the above-mentioned measurements being used to calculate flow rate a priori, or by means of a previously-determined calibration curve.

Alternatively, the heating may be carried out by applying power at a predetermined rate insufficient to dry said surface, a flow rate measurement being derived from a steady state temperature measured. Since this steady state temperature represents an equilibrium state in which the power applied balances the amount of energy being removed by evaporation, the flow rate can be derived based on the power applied and the temperature reached. For instance, the power can be applied at a predetermined rate, and the equilibrium temperature measured. Changes in flow rate will change this equilibrium temperature. Alternatively, the electrical power applied may be varied in order to maintain a predetermined steady state temperature, the power applied being used to derive the flow rate based on conservation of energy, or by using a calibration curve.

The invention also relates to a sensor system adapted to carry out any of the above-described methods.

This system includes a sensor comprising a surface adapted to receive a quantity of liquid thereupon, an electrical heating element arranged to be connected to a source of electrical power, as well as an arrangement for measuring temperature which, as above, may be a separate temperature sensor such as a thermistor, or may be an arrangement exploiting measurement of the resistance of the electrical heating element. The system also comprises a conduit adapted to collect a liquid sample and to transport it to said surface. This conduit may be a tube acting by capillary action or subject to a pumping action, a height difference or similar source of pressure head, or it may be a wicking fabric that transports liquid by capillary action.

Advantageously, the arrangement for measuring temperature is integrated with the electrical heating element as mentioned above, which results in a particularly compact system due to the absence of a separate thermistor or similar.

Advantageously, at least a part of said conduit may comprise a wicking fabric in fluid communication with the surface of the sensor, and which is arranged to be sandwiched between said sensor and a skin surface. A compact sensor system suitable for sweat rate measurements that can be worn on the skin can thus be constructed. This conduit advantageously extends around a peripheral wall of said sensor so as to form at least one fluid pathway leading to said surface.

Advantageously, the sensor system may further comprise a substantially impermeable cover arranged to at least partially seal said sensor system to said skin surface. This cover, which may be adhesive, serves to prevent undesired loss of liquid, ensuring that substantially all liquid reaches the surface of the sensor. The cover may be at least partially self-adhesive, so as to securely attach the sensor system to the skin of a wearer.

Advantageously, the sensor system further comprises an analysis sensor, such as a biological or chemical assay sensor, in fluid communication with said conduit and arranged such that said liquid passes through said analysis sensor before being received on the surface of the flow rate sensor. The flow rate sensor thus not only measures the flow rate of liquid passing through the analysis sensor, but also helps draw it through by creating a humidity gradient which enhances capillary flow in the conduit. Clogging of the analysis sensor with analytes is thus reduced or eliminated.

**Brief description of the drawings**

Further details of the invention will appear more clearly upon reading the description below, in connection with the following figures which illustrate:

- Fig. 1: a schematic diagram of a sensor as used in the method of the invention;
- Fig. 2: a schematic diagram of a variant of a sensor as used in the method of the invention;
- Fig. 3: a schematic graph representing the method as applied to volumetric measurements, not being part of the present invention;
- Fig. 4: a graph illustrating measurement data obtained with the method during calibrated volumetric measurements;

- Fig. 5: a graph of a calibration curve obtained from the data illustrated in figure 4;
- Fig. 6: a schematic graph representing an embodiment of the method of the invention as applied to flow rate measurements;
- Fig. 7: a graph of a calibration curve obtained using the method illustrated in figure 6;
- Fig. 8: a schematic graph representing a further embodiment of the method of the invention as applied to flow rate measurements;
- Fig. 9: a schematic representation of a microfluidic measurement system applying the method of the invention for measuring fluid flow rates;
- Fig. 10: a schematic representation of the sensor of figure 1 or 2, provided with several enhancements;
- Fig. 11: a schematic representation of a sweat rate sensor incorporating the sensor of figures 1, 2 or 10, and which apply the method of the invention.

## Embodiments of the invention

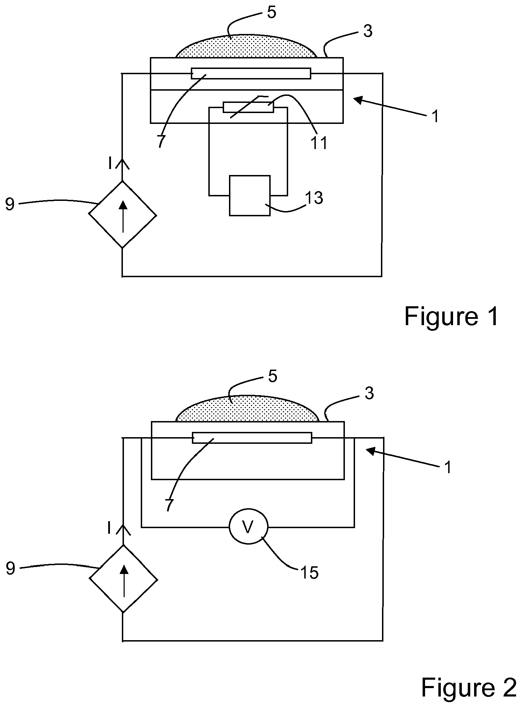

Figure 1 illustrates schematically the principle of the invention, which revolves around a particular method of using a sensor 1.

Sensor 1 comprises a surface 3 arranged to receive liquid 5 thereupon. This surface 3 may be flat, textured, smooth, or may comprise structures to aid in attracting and retaining liquid 5. For instance, hollows, capillary channels and so on may be provided thereupon. Furthermore, this surface 3 may be interchangeable so as to be replaceable in the case in which it becomes sufficiently coated with solid solutes from the liquid that it no longer functions correctly. In terms of materials, heat-conducting surfaces are preferred, such as metals (copper, brass, steel, aluminium, nickel, gold...), thermally-conductive ceramics (silicon carbide, aluminium nitride, sapphire, alumina), or similar.

Sensor 1 further comprises an electrical heating element 7, provided with electrical energy by means of a power source 9, which in this case is adapted to deliver a predetermined current I.

The sensor 1 also comprises a temperature sensor 11, illustrated here as a thermistor electrically connected to a resistance meter 13. Other forms of temperature sensor 11 are possible. In this setup, the electrical resistance of the electrical heating element 7 is known a priori as a function of temperature.

Figure 2 illustrates a variant of sensor 1, which uses the electrical heating element 7 itself not only as a source of heat, but also as a temperature sensor. Since the resistance of the electrical heating element 7 itself changes with temperature, by measuring the voltage drop across electrical heating element 7 by means of a voltmeter 15, its temperature can be deduced.

In each case, we know that the heating power supplied can be calculated as:

$$P = I^2R$$

Wherein P is heating power, I is current, and R is the electrical resistance of the electrical heating element. Power P can be integrated with respect to time to obtain the amount of heat energy Q applied, expressed in joules.

In the arrangement of figure 1, R may vary as a function of the temperature of the electrical heating element 7, and a calibration curve may be applied in function of the temperature measured by the temperature sensor 11 to account for this change.

In the arrangement of figure 2, the resistance of the electrical heating element 7 does not need to be known a priori, and the heating power supplied can simply be calculated as:

$$P = IV$$

Where P and I are defined as above, and V is the voltage drop across the electrical heating element 7 as measured by the voltmeter 15. Again, the heat energy Q applied can be calculated simply by integrating P with respect to time.

By knowing the rate of application of heat and/or the total heat applied, and the variation of temperature over time, the volume or the flow rate of the liquid 5 can be determined as described in more detail below. It should be noted that the present invention only applies to (unknown) flow rate measurements, however descriptions of volumetric measurements are also described herein even though they do not form part of the invention.

Contrary to the method described in US2014/208824, the present method operates the sensor in an evaporative mode. In other words, the temperature of the sensor 1 is raised to a sufficient degree to force evaporation of the liquid 5. It is this evaporation that is primarily exploited to determine the volume or flow rate of liquid 5, rather than just a simple temperature rise. While at any temperature above freezing in ambient conditions with a relative humidity under 100% evaporation will occur naturally, it is relatively insignificant compared to the forced evaporation used in the method of the invention, in which the absolute temperature of the sample is raised to at least 90% of the absolute-temperature (expressed in Kelvin) boiling point of the liquid (in the absence of solutes) at ambient pressure, by raising the surface 3 of the sensor 1 to at least this temperature. For an aqueous liquid, this corresponds to approximately 63°C, for an ethanol-based sample to at least 43°C, and for a typical white mineral oil, 243°C. However, absolute temperatures up to 100% or even 110% of the boiling point of the liquid 5 (considered without solutes) are also possible, particularly in the presence of large concentrations of solutes. It is also possible to use a temperature of at least 93%, at least 95%, at least 97%, or at least 99% of the absolute boiling temperature to the liquid, expressed in Kelvin. In the case of an aqueous liquid, these percentages correspond to approximately 74°C, 81°C, 89°C and 96°C respectively.

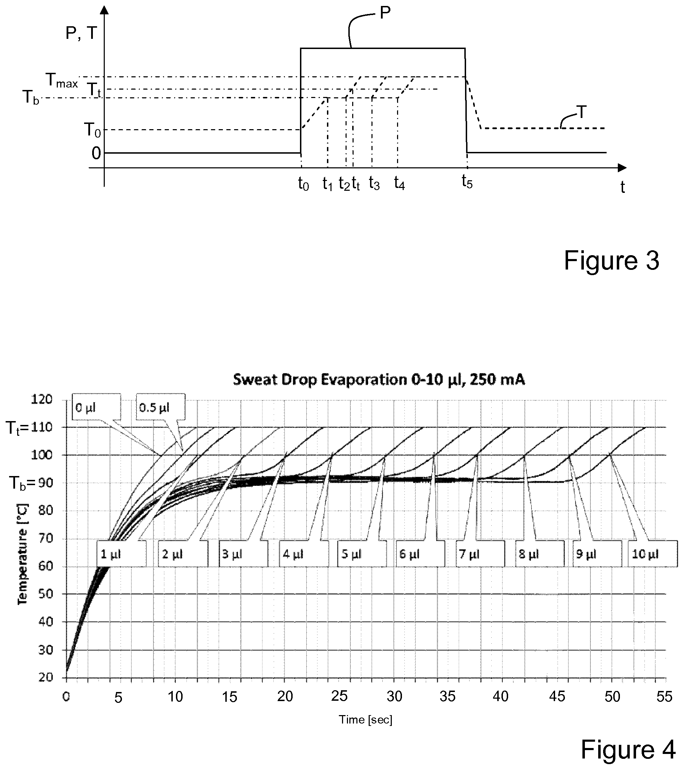

Firstly, a method for use of the sensor for volumetric measurements will be described. This method is described for information and is not to be construed as falling within the scope of the present invention. Such a method is useful for e.g. determining the volume of drops of liquid produced by a microfluidic device, a syringe or similar. This method is illustrated schematically by the idealised graph of figure 3, and heating power is supplied which is sufficient to heat the surface 3 to well above the boiling point of the liquid 5.

In this graph, the solid line represents the heating power applied, and the dashed lines represent simplified temperature profiles relating to three different liquid volumes 5. At time $t_0$, the temperature measured equals the initial temperature $T_0$, i.e. the temperature of the surroundings. At this point in time, heating power P is applied. Heating power P is typically constant during the heating phase, but does not have to be so, and is sufficient to raise the temperature of the liquid 5 to at least 90% of its boiling temperature as described above. Typically, the sensor is configured to reach an equilibrium surface temperature when dry (i.e. when no liquid is present) which is above the boiling point of the liquid 5 to be measured. This ensures that the sensor 1 is powerful enough to carry out the method correctly. As non-limiting examples, this dry equilibrium surface temperature can be at least 110% of the boiling temperature of the liquid 5, at least 115% thereof, or even at least 120% (or more) thereof.

Between $t_0$ and $t_1$, the heat predominantly causes the temperature of the liquid 5 to rise. As will be seen in more detail below, $t_1$ does not vary significantly in function of the volume of the sample, since a significant excess of heat is applied, and the latent heat of vaporisation of a liquid is significantly higher than its specific heat capacity. This can clearly be seen by comparing the values for water, namely 2257 kJ/kg latent heat of vaporisation and 4.2 kJ/kg.K specific heat capacity. In this case, the specific heat capacity is only around 0.2% of the latent heat of vaporisation.

From $t_1$ until $t_2$ (for the smallest liquid sample illustrated, i.e. the leftmost track on the graph, the times $t_3$ and $t_4$ corresponding to the same point for larger samples), the temperature T remains substantially constant at or near the boiling point $T_b$ of the liquid 5 while this latter evaporates. At $t_2$, the liquid has substantially all evaporated, and the temperature of the sensor 1 rises again until thermal equilibrium with the surroundings is reached at Tmax.

Finally, at $t_5$, the heating phase ends, and the temperature drops back to $T_0$ as the sensor 1 loses heat to its surroundings.

On the basis of the measurements taken (electrical power P, temperature T and time t), the amount of heat energy applied to the liquid 5 can be calculated since its specific heat capacity and latent heat of vaporisation are known.

Integrating the heating power applied between $t_0$ and $t_2$, $t_3$, or $t_4$ (as appropriate) to give the energy absorbed by the liquid, which then permits simple calculation of the mass, and hence the volume of liquid 5, by means of standard specific heat capacity and latent heat of vaporisation calculations that need not be reproduced here.

Figure 4 shows measurement data for evaporation of a sweat drop from 0 to 10 μL volume, with a constant current of 250 mA applied to the heating element 7. As can be seen from this data, the transition from the heating phase ($t_0$-$t_1$) is quite indistinct (and hence the various t points have not been indicated on this graph), which may be exacerbated further due to the presence of dissolved solutes in the liquid sample, such as salt in a sweat drop, and does not serve as a good basis for automated measurement. Furthermore, the exact moment that a given sample has fully evaporated ($t_2$, $t_3$, $t_4$...) is also indistinct, particularly in the case of smaller samples, rendering calculation by integration of the energy applied to the liquid 5 difficult to perform automatically with good accuracy.

In order to simplify determination of the liquid volume, a calibration curve can be created. This is simpler and often more accurate than calculation from first principles, since this does not require determining the times $t_1$ and $t_2$ on a smooth curve.

Firstly, a threshold temperature $T_t$ is defined which is ideally higher than the boiling temperature $T_b$ of the liquid but lower than the equilibrium temperature $T_{max}$. In the present example, $T_t$ is 110°C, about 5% above the absolute value of $T_b$ expressed in Kelvin. This ensures that the temperature curve crosses the threshold temperature $T_t$ at a distinct, easily-determinable point, at an easily-measurable time $T_t$.

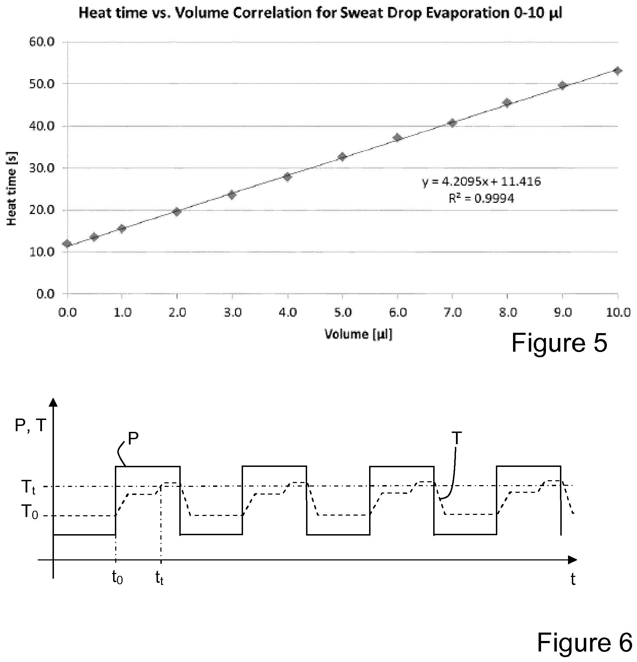

On the basis of the calibrated tests represented in figure 4, a calibration curve is calculated, which is represented in figure 5. This curve is linear to a high $R^2$ value, and can be represented as:

$$v = \frac{t - k_1}{k_2}$$

In which v is the volume of the liquid, t is the time taken from commencing heating at to to crossing the threshold temperature $T_t$ at time $t_t$ (i.e. t = $t_t$ - $t_0$), $k_1$ is a constant relating to the thermal lag of the sensor 1, and $k_2$ is a constant relating to the thermal properties of the liquid.

In the present example, a dry sensor takes 11.4 seconds to reach the threshold temperature, hence $k_1$=11.4, and $k_2$ is determined from the slope of the graph of figure 5, which is 4.2. Hence, in the present example, the calibration curve is reduced to:

$$v = \frac{t - 11.4}{4.2}$$

Both $k_1$ and $k_2$ will vary according to the ambient temperature, and $k_2$ will also vary according to the relative humidity, since it is a function of the rate of evaporation of the liquid. As a result, in order to increase the accuracy of the measurement, it may be desirable to generate a family of calibration curves, which are applied as a function of the ambient conditions as measured by other appropriate sensors (not illustrated). Alternatively, a single set of values of $k_1$ and $k_2$ can be used, with attendant loss of accuracy if the ambient conditions are significantly different. It should be noted that this calibration curve fits the data with extremely high precision, giving an $R^2$ value of 0.9994 with a linear fit. Other liquids may produce non-linear calibration curves.

Such a calibration curve-based calculation is significantly simpler to apply than a calculation a priori based on integration, and does not need a priori knowledge of specific heat capacities and latent heats of vaporisation of the liquid being measured. Furthermore, if the sensor is required to measure the volume of samples of different liquids, at least one calibration curve may be generated for each liquid, and the controller for the sensor 1 applies the correct calibration curve, either following a manual input, or an output of a sensor which determines which type of liquid is present. Also, it is worth noting that even if the temperature measured is not exactly the same as that of the surface 3 but is merely correlated thereto, a calibration curve method automatically takes this into account and any temperature difference between the surface 3 and the measured temperature does not need to be manually accounted for.

Since the method described above results in the complete evaporation of the sample, the sensor 1 is immediately ready to receive a further sample, which is not possible with the non-evaporative method of US2014/208824.

As mentioned above, over repeated measurements the presence of solid solutes in the liquid 5 may reduce the accuracy of the sensor 1, which will periodically require cleaning, or replacement of an interchangeable surface 3.

The same sensor 1 can also be used for continuous flow rate measurements according to the invention, when the sensor 1 is arranged in such a manner that fluid is introduced onto its surface 3 in a substantially continuous manner, for instance by capillary action, a microfluidic pump, or similar.

In one variant of such a flow rate measurement, the sensor 1 is operated as described above for discrete volume measurements, with periodic heating pulses which are sufficiently long and powerful to ensure that the surface 3 of the sensor 1 is dried during each pulse, and the power P can be determined as required to fulfil these conditions. This is illustrated on the graph of figure 6, which differs from that of figure 3 in that a single temperature curve T is illustrated, and multiple heating pulses are present, separated by sufficient time to allow the sensor 1 to return to substantially ambient temperature.

Since the volume of liquid 5 on the sensor 1 is being added to constantly, an integration-based calculation of flow rate from first principles is difficult, and hence a calibration-curve-based method is preferred.

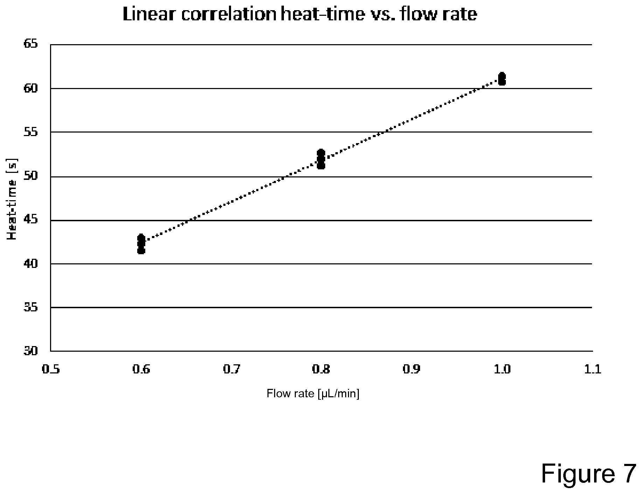

Figure 7 illustrates the calibration curve obtained with flow rates of 0.6, 0.8 and 1.0 $\mu$L/min, and the time from $t_0$ to $t_t$ (i.e. from the start of the pulse to the temperature reaching threshold temperature $T_t$) within each heating pulse. Each flow rate has three data points from three different tests, illustrating the excellent reproducibility of the system and method.

In a similar manner as before, the flow rate $\dot{v}$ is given in this example by

$$\dot{v} = \frac{t - k_3}{k_4}$$

wherein $\dot{v}$ is flow rate in $\mu$L/min, and $t = t_t - t_0$.

And by inserting the measured values for k3 and k4 obtained from the graph, we arrive at:

$$\dot{v} = \frac{t - 14}{47.2}$$

Again, the values of $k_3$ and $k_4$ can be determined for different sets of ambient conditions (temperature, relative humidity, pressure...).

It should be noted that the type of conduit used to bring liquid to the surface 3 of the sensor 1, and hence when determining the calibration curve, the sensor and all its associated elements should be set up as they will be used in reality. For instance, if a 1cm length of wicking material will be used as a conduit to bring fluid to the surface of the sample, then this should also be used during calibration, the calibrated flow rate of liquid (e.g. from a micropump, a syringe or similar) being applied to the wicking fabric as similarly as possible to how it will be applied in use. Also, it should be noted that the previous comments regarding exactly where the temperature is measured when using a calibration curve method apply equally to volumetric measurements, which perfectly illustrates the inherent practicality of such a method.

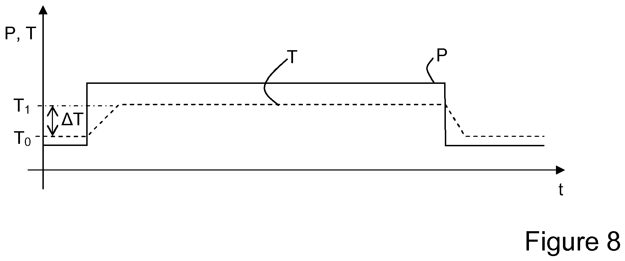

Figure 8 illustrates a further manner in which the sensor can be used as a flow sensor. In this variant, rather than applying power P in a pulsed fashion, it is applied constantly. In this variant, the surface 3 of the sensor remains wet, and the temperature attained $T_1$ and/or the change in temperature $\Delta T$ at equilibrium varies in function of the flow rate, and the power P is predetermined in function of the expected flow rate and the properties of the liquid being measured.

Since a higher flow rate results in more cooling of the sensor 1, higher flow rates result in a lower equilibrium temperature $T_1$ and vice-versa. It should be noted that the equilibrium temperature in this embodiment is obtained with the surface 3 being wet, and is not a dry equilibrium temperature as described in relation to figure 3.

From this value $T_1$ and/or the change from the initial temperature To, the flow rate can be determined a priori or by means of a calibration curve in a manner similar to that described above.

As an alternative to a fixed, predetermined power P, this latter can be adjusted to ensure that the surface 3 of the sensor 1 remains wet, for instance by being reduced if the temperature rises suddenly in such a manner as to indicate that the sensor 1 is dry. A further variant involves adjusting power P to maintain a predetermined temperature $T_1$. In all of these cases, the flow rate of liquid can be determined either a priori by calculation or by means of calibration curves based on the values of P and $T_1$ (or $\Delta T$) as appropriate, optionally taking into account the ambient temperature and/or pressure and/or relative humidity. In such a case, appropriate dedicated sensors can be provided.

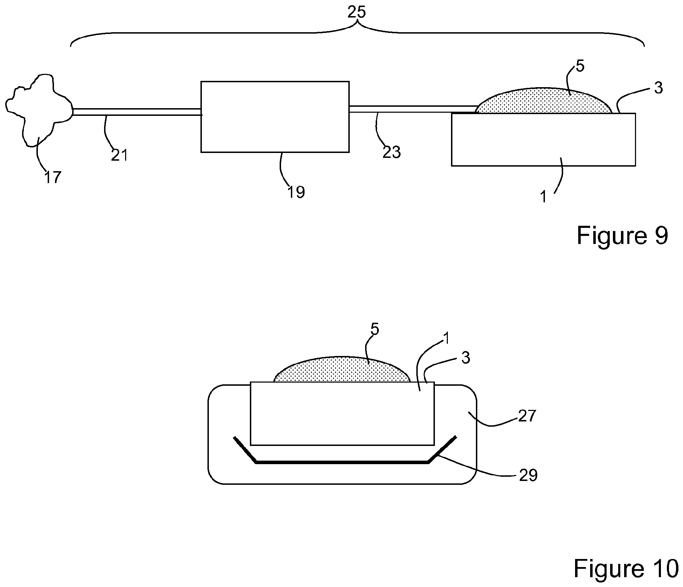

Figure 9 illustrates a further application of the sensor 1 according to any of the above-mentioned embodiments, as part of a microfluidic measurement system 25 based on the method of the invention. It should be noted that any system incorporating the sensor 1 and applying a method according to the invention can be referred to generically as a "sensor system".

This system 25 takes liquid from a source 17, which may be for instance a sample of liquid in a test tube, the sweating skin of a person, or any other source, and carries it via a first conduit 21 to an input of an analysis sensor 19. This sensor may be of any convenient type, e.g. adapted to perform a biological or chemical assay, to detect particular chemical species, or similar. The output of the analysis sensor communicates with the surface 3 of the sensor 1 via a second conduit 23, so as to create a liquid pathway from the source 17, via the analysis sensor 19, to the sensor 1. These conduits may, for instance, be one or more capillary tubes, a wicking fabric, or similar. In the case of a wicking fabric, this can be enclosed in an impermeable sheath to prevent lateral evaporation from its structure.

As a result of the construction of this system 25, the sensor 1 of the invention not only provides a measurement of the flow rate of liquid through the analysis sensor 19 by means of its accompanying circuitry (not illustrated in this figure), but also helps to draw the liquid through it without the need for a microfluidic pump. Conventionally, in the absence of a pump, the measurements provided by the analysis sensor 19 will start to drift since the liquid is not being continually and positively drawn through and thus the analysis sensor 19 is not flushed through, and hence cannot clear itself properly. This leads to buildups of analytes in the analysis sensor 19.

Figure 10 illustrates a sensor 1 according to the invention, again according to any of the above-mentioned embodiments, which in this case embedded in a low thermal conductivity material 27 such as a ceramic, a glass, a glass-ceramic, a polymer or similar, so as to reduce heat loss and to ensure that all of the power P applied thereto is directed towards the liquid 5 on the exposed surface 3 of the sensor 1. Additionally or alternatively, an infrared reflector 29 may be provided so as to minimise heat loss in the direction away from the surface 3 for receiving the liquid 5.

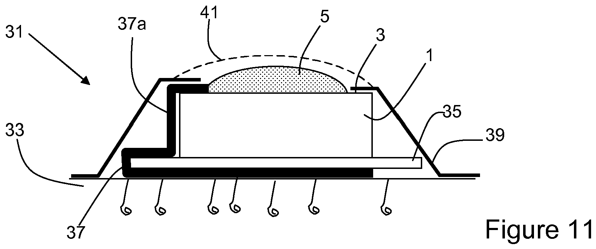

Figure 11 illustrates a sweat rate sensor 31 incorporating a sensor 1 according to the invention, intended to be placed upon the skin 33 of a wearer.

The sensor 1 is situated upon an insulating element 35, adapted to prevent the heat of the sensor 1 from heating the skin 33 to the degree that it becomes uncomfortable. This insulating element 35 may be of plastic, ceramic, glass, glass-ceramic, fabric, or a combination thereof. Furthermore, an infrared reflector 29, as described above, may also be provided between the sensor 1 and the skin 33.

In order to collect sweat from the skin 33 and transport it to the exposed surface 3 of the sensor 1, a wicking fabric element 37 is provided between the insulating element 35 and the skin 33, and is in contact with the latter. The wicking fabric element 37 comprises at least one wicking pathway 37a leading around the sides of the sensor 1 to its exposed surface 3, so as to transport excreted sweat thereto. This construction minimises evaporative loss from the wicking fabric element 37, and results in a particularly compact structure. Alternatively, the wicking fabric element 37 may extend to the side of the sensor 1, which results in a less compact sweat rate sensor 31 in terms of the area of skin it occupies.

In order to prevent evaporative loss of sweat on its way to the surface 3, a substantially impermeable cover 39 at least partially seals the sweat rate sensor 31 to the skin 33. This cover 39 may for instance be made of a flexible polymer, silicone rubber or similar, and may be omitted if sweat losses are trivial.

The sweat rate sensor 31 can be affixed to the skin of a person e.g. by means of a strap, or an adhesive support similar to that used for venous catheters, but sized to surround the sweat rate sensor 31. Such an adhesive support may also serve as the cover 39, so as to attach the sweat rate sensor 31 to the skin of a wearer.

Once sweat produced by the sweat glands in the skin 33 has reached the surface 3 of the sensor 1, this latter can be operated according to one of the methods described above so as to provide measurements of the rate of sweating. The capillary pressure caused by the heated surface 3 and the continuous evaporation of the sweat 5 continuously draws the sweat from the skin 33 to the surface 3.

Furthermore, in order to prevent air movements (originating e.g. from wind or resulting from movements of the wearer) from affecting the rate of evaporation of the liquid 5, an optional semipermeable membrane 41 may be provided. Such a membrane may be made of Gore-Tex® (porous polytetrafluoroethylene) or similar material which allows water vapour to pass through, but is substantially impermeable to liquid water. As a result, forced evaporative cooling is reduced, and the evaporation rate is rendered substantially independent of air flows adjacent to the sensor 1. This membrane 41 may be bonded to the cover 39, or may be bonded to the sensor 1, e.g. to its surface 3 or to its outer wall. A passage for the conduit 37 may be provided if needed.

This sweat rate sensor 31 may also comprise an analysis sensor interposed in the fluid pathway between the skin and the surface 3, in the manner disclosed in reference to figure 9.

Although the invention has been described in terms of particular embodiments, various modifications are possible without departing from the scope of the invention as defined in the appended claims.

**Claims**

1. Method for liquid measurements relating to unknown flow rates, comprising:

   - providing a sensor (1) comprising a surface (3) arranged to receive a liquid (5) thereupon, an electrical heating element (7) arranged to heat said surface (3), and an arrangement (11, 13; 7, 15) for measuring a temperature of said surface (3);
   - receiving liquid (5) on said sensor surface (3);
   - heating said surface (3) by means of said electrical heating element (7) at a known rate (P) while taking a plurality of temperature measurements (T) thereof in function of time (t), said heating being at an absolute temperature of at least 90% of the boiling point ($T_b$) of said liquid (5) under ambient pressure;
   - deriving a flow rate measurement of an unknown flow rate of said liquid (5) from said electrical heating rate (P) and said temperature measurements (T).

2. Method according to claim 1, wherein said electrical heating element (7) is provided with a predetermined electrical current (I) from a power source (9).

3. Method according to one of claims 1 or 2, wherein said temperature is measured by measuring the electrical resistance of said electrical heating element (7).

4. Method according to claim 3, wherein said electrical resistance is measured by providing a known current (I) to said electrical heating element while measuring the voltage drop thereacross, the resistance being calculated by Ohm's Law.

5. Method according to any preceding claim, wherein said heating is carried out as a series of heating pulses of predetermined duration.

6. Method according to claim 5, wherein said predetermined duration is sufficiently long to dry said surface.

7. Method according to any of claims 1-4, wherein said heating is carried out by applying power at a predetermined rate (P) insufficient to dry said surface, said flow rate measurement being derived from a steady state temperature (T) measured.

8. Method according to any of claims 1-4, wherein said heating is carried out by applying electrical power at a rate (P) insufficient to dry said surface, wherein electrical power is applied to maintain a predetermined steady state temperature (T), said flow rate measurement being derived from a measurement of said electrical power (P) applied.

9. Sensor system (25; 31) adapted to carry out the method of any preceding claim, comprising:

   - a sensor (1) comprising a surface (3) adapted to receive a quantity (5) of liquid thereupon, said sensor (1) comprising an electrical heating element (7) arranged to be connected to a source of electrical power (9) and an arrangement for measuring temperature (11, 13; 7; 15);
   - a conduit (21, 23; 37) adapted to collect a liquid sample and to transport said liquid sample to said surface (3).

10. Sensor system (25; 31) according to claim 9, wherein said arrangement for measuring temperature (7, 15) comprises said electrical heating element (7).

11. Sensor system (25; 31) according to one of claims 9 or 10, wherein at least a part of said conduit (21, 23; 37) comprises a wicking fabric arranged to be sandwiched between said sensor (19) and a skin surface (33) of a wearer.

12. Sensor system (25; 31) according to claim 11, wherein said conduit (21, 23; 37) extends around a peripheral wall of said sensor (1) so as to form at least one fluid pathway leading to said surface (3).

13. Sensor system (25; 31) according to one of claims 11 or 12, further comprising a substantially impermeable cover (39) arranged to at least partially seal said sensor system (25; 31) to said skin surface (33).

14. Sensor system (25; 31) according to claim 13, wherein said cover (39) is at least partially self-adhesive.

**15.** Sensor system (25; 31) according to one of claims 9-14, further comprising an analysis sensor (19) in fluid communication with said conduit and arranged such that said liquid passes through said analysis sensor (19) on its way to the surface (3) of the sensor (1).

**Patentansprüche**

**1.** Verfahren für Flüssigkeitsmessungen im Zusammenhang mit unbekannten Flussraten, umfassend:

- Bereitstellen eines Sensors (1), umfassend eine Oberfläche (3), die zum Aufnehmen einer Flüssigkeit (5) darauf angeordnet ist, ein elektrisches Heizelement (7), das zum Heizen der Oberfläche (3) angeordnet ist, sowie eine Anordnung (11, 13; 7, 15) zum Messen einer Temperatur der Oberfläche (3);
- Aufnehmen von Flüssigkeit (5) auf der Sensoroberfläche (3) ;
- Heizen der Oberfläche (3) mittels des elektrischen Heizelements (7) mit einer bekannten Rate (P) bei gleichzeitigem Aufnehmen mehrerer Temperaturmessungen (T) davon als Funktion der Zeit (t), wobei das Heizen bei einer absoluten Temperatur von mindestens 90% des Siedepunkts ($T_b$) der Flüssigkeit (5) unter Umgebungsdruck erfolgt;
- Ableiten einer Flussratenmessung einer unbekannten Flussrate der Flüssigkeit (5) aus der elektrischen Heizrate (P) und den Temperaturmessungen (T).

**2.** Verfahren nach Anspruch 1, wobei das elektrische Heizelement (7) mit einem vorbestimmten elektrischen Strom (I) aus einer Leistungsquelle (9) versorgt wird.

**3.** Verfahren nach einem der Ansprüche 1 oder 2, wobei die Temperatur durch Messen des elektrischen Widerstands des elektrischen Heizelements (7) gemessen wird.

**4.** Verfahren nach Anspruch 3, wobei der elektrische Widerstand durch Zuführen eines bekannten Stroms (I) zu dem elektrischen Heizelement bei gleichzeitigem Messen des Spannungsabfalls über dieses gemessen wird, wobei der Widerstand anhand des Ohmschen Gesetzes berechnet wird.

**5.** Verfahren nach einem der vorhergehenden Ansprüche, wobei das Heizen als eine Serie von Heizimpulsen einer vorbestimmten Dauer durchgeführt wird.

**6.** Verfahren nach Anspruch 5, wobei die vorbestimmte Dauer ausreichend lang ist, um die Oberfläche zu trocknen.

**7.** Verfahren nach einem der Ansprüche 1-4, wobei das Heizen durch Anwenden von Leistung mit einer vorbestimmten Rate (P) durchgeführt wird, die nicht ausreicht, um die Oberfläche zu trocknen, wobei die Flussratenmessung aus einer gemessenen stabilen Zustandstemperatur (T) abgeleitet wird.

**8.** Verfahren nach einem der Ansprüche 1-4, wobei das Heizen durch Anwenden von elektrischer Leistung mit einer Rate (P) durchgeführt wird, die nicht ausreicht, um die Oberfläche zu trocknen, wobei die elektrische Leistung angewendet wird, um eine vorbestimmte stabile Zustandstemperatur (T) aufrechtzuerhalten, wobei die Flussratenmessung von einer Messung der angewendeten elektrischen Leistung (P) abgeleitet wird.

**9.** Sensorsystem (25; 31), angepasst zum Durchführen des Verfahrens nach einem der vorhergehenden Ansprüche, umfassend:

- einen Sensor (1), umfassend eine Oberfläche (3), die zum Aufnehmen einer Quantität (5) von Flüssigkeit darauf angepasst ist, wobei der Sensor (1) ein elektrisches Heizelement (7) umfasst, das dazu angeordnet ist, mit einer Quelle von elektrischer Leistung (9) verbunden zu werden, und auch eine Anordnung zum Messen von Temperatur (11, 13; 7; 15) umfasst;
- einen Kanal (21, 23; 37), der zum Sammeln einer Flüssigkeitsprobe und zum Transportieren der Flüssigkeitsprobe zur Oberfläche (3) angepasst ist.

**10.** Sensorsystem (25; 31) nach Anspruch 9, wobei die Anordnung zum Messen von Temperatur (7, 15) das elektrische Heizelement (7) umfasst.

**11.** Sensorsystem (25; 31) nach einem der Ansprüche 9 oder 10, wobei zumindest ein Teil des Kanals (21, 23; 37) ein Kapillargewebe umfasst, das angeordnet ist, um zwischen dem Sensor (19) und einer Hautoberfläche (33) eines Trägers positioniert zu werden.

**12.** Sensorsystem (25; 31) nach Anspruch 11, wobei sich der Kanal (21, 23; 37) rund um eine periphere Wand des Sensors (1) erstreckt, um zumindest eine Flüssigkeitspassage zu bilden, der zu der Oberfläche (3) führt.

**13.** Sensorsystem (25; 31) nach einem der Ansprüche 11 oder 12, ferner umfassend eine im Wesentlichen undurchlässige Abdeckung (39), angeordnet, um zumindest teilweise das Sensorsystem (25; 31) gegen die Hautoberfläche (33) abzudichten.

**14.** Sensorsystem (25; 31) nach Anspruch 13, wobei die Abdeckung (39) zumindest teilweise selbstklebend ist.

**15.** Sensorsystem (25; 31) nach einem der Ansprüche 9-14, ferner umfassend einen Analysesensor (19) in Fluidverbindung mit dem Kanal und so angeordnet, dass die Flüssigkeit auf ihrem Weg zur Oberfläche (3) des Sensors (1) den Analysesensor (19) passiert.

**Revendications**

**1.** Procédé pour des mesures de liquide relatives à des débits inconnus, le procédé comprenant les étapes de :

- fournir un capteur (1) comprenant une surface (3) agencée pour y recevoir un liquide (5), un élément chauffant électrique (7) agencé pour chauffer ladite surface (3), et un agencement (11, 13 ; 7, 15) pour mesurer une température de ladite surface (3) ;
- recevoir un liquide (5) sur ladite surface de capteur (3) ;
- chauffer ladite surface (3) au moyen dudit élément chauffant électrique (7) à un taux connu (P) tout en prenant une pluralité de mesures de température (T) correspondantes en fonction du temps (t), ledit chauffage étant à une température absolue d'au moins 90 % du point d'ébullition ($T_b$) dudit liquide (5) sous pression ambiante ;
- obtenir une mesure de débit d'un débit inconnu dudit liquide (5) à partir dudit régime de chauffage électrique (P) et desdites mesures de température (T).

**2.** Procédé selon la revendication 1, dans lequel ledit élément chauffant électrique (7) reçoit un courant électrique (I) prédéterminé en provenance d'une source d'alimentation (9).

**3.** Procédé selon la revendication 1 ou 2, dans lequel ladite température est mesurée en mesurant la résistance électrique dudit élément chauffant électrique (7).

**4.** Procédé selon la revendication 3, dans lequel ladite résistance électrique est mesurée en fournissant un courant connu (I) audit élément chauffant électrique tout en mesurant la chute de tension entre ses bornes, la résistance étant calculée par la loi d'Ohm.

**5.** Procédé selon l'une quelconque des revendications précédentes, dans lequel ledit chauffage est réalisé sous la forme d'une série d'impulsions de chauffage de durée prédéterminée.

**6.** Procédé selon la revendication 5, dans lequel ladite durée prédéterminée est suffisamment longue pour sécher ladite surface.

**7.** Procédé selon l'une quelconque des revendications 1 à 4, dans lequel ledit chauffage est réalisé en appliquant une puissance à un régime prédéterminé (P) insuffisant pour sécher ladite surface, ladite mesure de débit étant obtenue à partir d'une température à l'état stable (T) mesurée.

**8.** Procédé selon l'une quelconque des revendications 1 à 4, dans lequel ledit chauffage est réalisé en appliquant une puissance électrique à un taux (P) insuffisant pour sécher ladite surface, ladite puissance électrique étant appliquée pour maintenir une température à l'état stable (T) prédéterminée, ladite mesure de débit étant obtenue à partir d'une mesure de ladite puissance électrique (P) appliquée.

**9.** Système capteur (25 ; 31) adapté pour réaliser le procédé selon l'une quelconque des revendications précédentes, le système capteur comprenant :

- un capteur (1) comprenant une surface (3) adaptée pour y recevoir une quantité (5) de liquide, ledit capteur (1) comprenant un élément chauffant électrique (7) agencé pour être connecté à une source de puissance électrique (9) et un agencement de mesure de température (11, 13 ; 7 ; 15) ;
- un conduit (21, 23 ; 37) adapté pour collecter un échantillon de liquide et pour transporter ledit échantillon de liquide vers ladite surface (3).

**10.** Système capteur (25 ; 31) selon la revendication 9, dans lequel ledit agencement de mesure de température (7, 15) comprend ledit élément chauffant électrique (7).

**11.** Système capteur (25 ; 31) selon l'une des revendications 9 et 10, dans lequel au moins une partie dudit conduit (21, 23 ; 37) comprend un tissu mèche agencé pour être intercalé entre ledit capteur (19) et une surface de peau (33) d'un porteur.

**12.** Système capteur (25 ; 31) selon la revendication 11, dans lequel ledit conduit (21, 23 ; 37) s'étend autour d'une paroi périphérique dudit capteur (1) de manière à former au moins un passage de fluide conduisant vers ladite surface (3).

**13.** Système capteur (25 ; 31) selon l'une des revendications 11 et 12, comprenant en outre un couvercle sensiblement imperméable (39) agencé pour sceller au moins en partie ledit système capteur (25 ; 31) sur ladite surface de peau (33).

**14.** Système capteur (25 ; 31) selon la revendication 13, dans lequel ledit couvercle (39) est, au moins en partie, autoadhésif.

**15.** Système capteur (25 ; 31) selon l'une des revendications 9 à 14, comprenant en outre un capteur d'analyse (19) en communication fluidique avec ledit conduit et agencé de sorte que ledit liquide traverse ledit capteur d'analyse (19) sur son chemin vers la surface (3) du capteur (1).

Figure 1

Figure 2

Figure 3

Sweat Drop Evaporation 0-10 μl, 250 mA

Figure 4

Figure 5

Figure 6

Linear correlation heat-time vs. flow rate

Figure 7

Figure 8

Figure 9

Figure 10

Figure 11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2014208824 A
- US 2003215334 A
- WO 0156436 A
- WO 2017019602 A

### Non-patent literature cited in the description

- **IMHOF et al.** Closed-chamber transepidermal water loss measurement: microclimate, calibration and performance. *Int J Cosmet Sci.,* April 2009, vol. 31 (2), 97-118
- Continuous micro liquid delivery by evaporation on a gradient-capillary microstructure surface. **XIAOZE DU et al.** Journal of Microengineering. Institute of Physics Publishing, vol. 21, 95004
- A microfluidic device based on an evaporation-driven micropump. **NIE CHUAN et al.** Biomedical Devices. Kluwer, vol. 17, 1-12